**Europäisches Patentamt**

**European Patent Office**

**Office européen des brevets**

(19)

(11) Veröffentlichungsnummer : **0 366 950 B1**

# (12) EUROPÄISCHE PATENTSCHRIFT

(45) Veröffentlichungstag der Patentschrift :
**16.12.92 Patentblatt 92/51**

(21) Anmeldenummer : **89118276.8**

(22) Anmeldetag : **03.10.89**

(51) Int. Cl.$^5$ : **A61M 1/16**

(54) Hämodialysevorrichtung mit Entlüftungseinrichtung.

(30) Priorität : **07.10.88 DE 3834126**

(43) Veröffentlichungstag der Anmeldung :
**09.05.90 Patentblatt 90/19**

(45) Bekanntmachung des Hinweises auf die
Patenterteilung :
**16.12.92 Patentblatt 92/51**

(84) Benannte Vertragsstaaten :
**DE ES FR GB IT SE**

(56) Entgegenhaltungen :
**WO-A-81/01800**
**DE-A- 3 418 434**
**DE-C- 2 838 414**

(73) Patentinhaber : **Fresenius AG**
**Gluckensteinweg 5**
**W-6380 Bad Homburg v.d.H. (DE)**

(72) Erfinder : **Polaschegg, Hans Dietrich, Dr. Phys.**
**Grünwiesenweg 9**
**W-6370 Oberursel (DE)**

(74) Vertreter : **Dr. Fuchs, Dr. Luderschmidt Dr.**
**Mehler, Dipl.-Ing Weiss Patentanwälte**
**Abraham-Lincoln-Strasse 7**
**W-6200 Wiesbaden (DE)**

EP 0 366 950 B1

## Beschreibung

Die Erfindung betrifft eine Hämodialysevorrichtung gemäß dem ober begriff des Anspruch 1

Die große Zahl der nierenkranken Patienten und die mit ihrer Behandlung verbundenen Kosten stellen eine nicht unbeträchtliche Belastung der Volkswirtschaft dar. Durch zahlreiche Maßnahmen wird daher angestrebt, die Kosten dieser Dialysebehandlung zu senken. Da im Laufe der letzten 25 Jahre, in denen die Behandlung der chronischen Niereninsuffizienz zur Routine geworden ist, die reinen Materialkosten, d.h. die anteiligen Kosten der Hämodialysegeräte sowie die Kosten der Einmalartikel (Hämodialysator, Blutschlauchsystem, Dialysekonzentrate, Kanülen und dergl.), zum Unterschied zu den Arzneimittelkosten und Behandlungskosten ständig gesunken sind, stellen heute die Fixkosten der Gebäude und der dazugehörigen Einrichtungen sowie vor allem die Personalkosten den Hauptteil der Behandlungskosten dar.

Es wird daher angestrebt, die für eine Behandlung notwendige Zeit, die sich aus Maschinen-Vorbereitungszeit, Behandlungszeit des Patienten sowie Maschinen-Nachbereitungszeit zusammensetzt, zu verkürzen. Da aufgrund neuerer Erkenntnisse bei einem großen Prozentsatz der Patienten die Behandlungszeit von ehemals 8 Stunden (heute durchschnittlich 4-5 Stunden) auf 2-3 Stunden verkürzt werden kann, stellt die Zeit, die zur Vorbereitung der Maschine benötigt wird, einen immer größeren Anteil dar. Insofern soll der hierfür nötige Arbeitsaufwand durch geeignete konstruktive Maßnahmen möglichst verringert werden.

Ein wesentlicher Arbeitsaufwand stellt das luftfreie Füllen des extrakorporalen Blutkreislaufs mit physiologischer Kochsalzlösung bzw. des Dialysierflüssigkeitskreislaufs mit Dialysierflüssigkeit dar.

Da üblicherweise die Dialyse im Gegenstrom betrieben wird, sieht der Stand der Technik zunächst die blutseitige Füllung des Dialysators vor, wobei die Kochsalzlösung dem tiefer gelegenen Anschluß zugeführt wird, so daß dadurch die in der Blutkammer des Dialysators vorhandene Luft durch den oben gelegenen Anschluß verdrängt wird und dort entweichen kann. Anschließend wird der üblicherweise in einer drehbaren Halterung befestigte Dialysator um 180° gedreht und dialysierflüssigkeitsseitig ebenfalls von unten nach oben gefüllt.

Dieser Vorgang kann auch in umgekehrter Reihenfolge vorgenommen werden, d.h. zunächst wird die Dialysierflüssigkeitsseite und anschließend die Blutseite des Dialysators gefüllt. Im übrigen ist bei einigen Dialysatortypen eine bestimmte Fülloperation aus technischen Gründen vorgeschrieben.

Der oben beschriebene Vorgang wird bei der eingangs erwähnten Vorrichtung, die von der Anmelderin unter der Bezeichnung "A 2008" vertrieben wird oder in der DE-OS 28 38 414 beschrieben ist, manuell durchgeführt und eignet sich nicht ohne größeren Aufwand zur Automatisierung, da hierzu Einrichtungen zur Beobachtung der Luftfreiheit der ausströmenden Flüssigkeit sowie Einrichtungen zum Drehen des Dialysators erforderlich wären.

Deshalb hat man bei einem Dialysegerät mit der Bezeichnung "Seratron" ein Verfahren gewählt, bei dem die Blutpumpe mit einer Einrichtung zur Umkehrung der Förderrichtung versehen wurde. Diese Einrichtung erlaubt es, den Dialysator sowie das Blutschlauchsystem bei feststehendem Dialysator luftfrei von unten her zu füllen. Die Blutpumpe fördert dabei während des Füllvorgangs die Flüssigkeit entgegen der im Dialysierbetrieb vorgesehenen Richtung von unten nach oben. Dieses Verfahren funktioniert und wird inzwischen auch bei dem Hämodialysegerät Centrysystem 3 der Firma Cobe eingesetzt, ist jedoch mit folgendem Sicherheitsrisiko behaftet, das zusätzliche Überwachungseinrichtungen erforderlich macht.

Zur Vermeidung einer Luftinfusion im Fehlerfall muß - auch für den Fall eines beliebigen ersten Fehlers - eine Umkehrung der Förderrichtung der Blutpumpe während der Dialysebehandlung ausgeschlossen sein. Dies gilt auch für den Fall einer manuellen Betätigung im Notfall.

Bei Hämodialysegeräten, die für eine manuelle Füllung, d.h. Drehen des Dialysators, konstruiert sind, wird dies durch Pumpenkonstruktionen gewährleistet, die eine Umkehrung der Drehrichtung ausschließen. Für derartige Zwecke kommen Pumpen mit gleichstrombetriebenen Motoren und/oder mechanischen Rücklaufsperren zum Einsatz.

Führt man nun das Entlüften des Dialysators mit Hilfe einer Pumpe mit umkehrbarer Drehrichtung durch, so sind diese oben erwähnten einfachen Maßnahmen ersichtlich nicht mehr einsetzbar. Es ist daher eine Überwachungseinrichtung erforderlich, die einerseits die Drehrichtung der Pumpe überwacht, andererseits den Betriebszustand des Hämodialysegerätes erfaßt (beispielsweise überprüft, ob Blut oder Kochsalzlösung im Schlauchsystem ist). Die Funktion dieses Überwachungssystems muß ihrerseits überprüfbar sein, was zu einer sicherheitstechnisch aufwendigen Lösung führt.

Es wurden auch andere Versuche unternommen, das Schlauchsystem und den Dialysator auf einfache Weise zu füllen und zu entlüften.

So ist in der DE-OS 34 18 434 ein Verfahren zum Füllen und Entlüften des Dialysators beschrieben, bei dem das gesamte Schlauchsystem und der Dialysator zunächst unter Vakuum von Luft befreit wird und anschließend mit Flüssigkeit gefüllt wird. Hierzu muß der Dialysator auf der Dialysierflüssigkeitsseite verschlossen sein. Desgleichen dürfen die Poren der Membran nicht mit einer befeuchtenden Flüssigkeit gefüllt sein, da ansonsten das auf der Blutseite angelegte Vakuum nicht die Dialysierflüssigkeitskammer des Dialysators entlüften kann. Sofern also eine befeuchtete Membran eingesetzt wird, muß der Dialysator wiederum manuell gedreht werden, um ein sicheres Entlüften sicherzustellen.

Aus der US-PS 44 11 866 sowie aus der PCT-Schrift WO 84/03229 sind jeweils Dialysatoren bekannt, die betriebsseitig zumindest teilweise mit Flüssigkeit gefüllt worden sind und dann bettseitig unmittelbar einsetzbar sind. Hierzu müssen diese gefüllten Dialysatoren lediglich auf der Dialysatseite mit den Dialysatleitungen verbunden werden. Dieser Verbindungsvorgang führt jedoch unweigerlich zum Einschleppen von Luft in den Dialysator, d.h. auch hier müssen wiederum besondere Vorkehrungen durch das Personal getroffen werden, um ein vollständiges Entlüften des Dialysators herzustellen. Insofern ist auch hier kein einfaches, automatisiertes Füllen und Entlüften des Dialysators möglich.

Aus der WO-A-81/01800 ist eine Hämodialysevorrichtung der eingang erwähuten Art Bekannt, bei der Dialysatzu- und ablauf mittels zweier 3-Ventilanordnungen verlauscht werden Können. Solche Anordnungen sind technisch kompliziert und gegen Ausfall problematisch.

Der Erfindung liegt daher die Aufgabe zugrunde, eine Dialysevorrichtung der eingangs erwähnten Art zur Verfügung zu stellen, bei der das Füllen und Entlüften des Dialysators mit einem möglichst geringen sicherheitstechnischen Aufwand durchgeführt werden kann.

Die Lösung der Aufgabe erfolgt durch, die Kennzeichnen der Merkmale des Anspruch 1.

Erfindungsgemäß wird der Dialysator feststehend angeordnet und blutseitig von unten nach oben und gemäß einer ersten Ausführungsform dialysierflüssigkeitsseitig von oben nach unten, d.h. im Gegenstrom, durchströmt. Parallel zur Dialysierflüssigkeitsseite wird eine Rezirkulationspumpe angeordnet und es wird am dialysierflüssigkeitsseitigen Ausgang des Dialysators ein Luftabscheider angeschlossen. Zum Füllen des Dialysators wird nun die parallel zur Dialysierflüssigkeitsseite liegende Rezirkulationspumpe mit einer Förderrate betrieben, die höher ist als die vom Hämodialysegerät, also von der Dialysierflüssigkeitspumpe gelieferte Dialysierflüssigkeitsrate. Es kommt daher zur Umkehrung der Strömungsrichtung durch Rezirkulation von Dialysierflüssigkeit durch die dialysatseitige Kammer des Dialysators. Diese Dialysierflüssigkeit durchströmt den Dialysator nunmehr von unten nach oben, wobei die überschüssige Luft nach oben entweicht, durch die Rezirkulationspumpe zum Luftabscheider und dort mit der ausgeschiedenen Dialysierflüssigkeit zum Abfluß gefördert wird, während der rezirkulierende Flüssigkeitsanteil von Luft befreit weiterhin den Dialysator durchströmt.

Bei offenen Dialysevorrichtungssystemen reicht daher eine Entlüftungseinrichtung in Form eines Mischgefäßes aus, bei dem am oberen Ende das auszuscheidende Dialysierflüssigkeits/Luft-Gemisch ausgeschieden wird, während in das untere Ende die Rezirkulationsleitung und die die auszuscheidende Dialysierflüssigkeit fördernde Leitung einmünden.

Bei bilanzierenden Hämodialysevorrichtungen, d.h. bei geschlossenen Systemen, kann die Luftabscheideeinrichtung an ihrem oberen Ende einen Auslaß aufweisen, der mit einer Entlüftungspumpe versehen ist, die einen ausreichenden Unterdruck in dem Entlüftungsgefäß zum Entziehen der Luft erzeugen kann. Eine solche Einrichtung ist dann in entsprechender Weise mit einer Niveauregulierungseinrichtung versehen, die bei Unterschreiten des Niveaus die Entlüftungseinrichtung betätigt.

Nach dem Füllen und Entlüften kann die Rezirkulationspumpe einfach abgeschaltet werden. Dies ist ein Vorgang, der einfach überwacht bzw. sichergestellt werden kann. Darüber hinaus stellt eine Umkehrung der Förderrichtung der Rezirkulationspumpe auf der Dialysierflüssigkeitsseite keine akute Gefährdung des Patienten dar, da hierdurch nur ein Übergang vom Gegenstrom- zum Mitstromverfahren erfolgt, was eine Verminderung der Effektivität (Clearance) des Dialyseverfahrens bewirkt.

Die erfindungsgemäße Hämodialysevorrichtung kann jedoch nicht nur während des Entlüftens, sonderngemäß einem weiteren übergeordneten Erfindungsgedanken - auch während des üblichen Dialysebetriebs zum Einsatz kommen. Bekanntlich kann durch die Rezirkulation von Dialysierflüssigkeit, wie sie beispielsweise in dem US-Patent 35 27 700 oder der Monographie von Nosé "The artificial kidney", Band I, Verlag The C.V. Mosby Company, St. Louis, 1969, Seite 134 und 135 oder in der DE-OS 36 20270 beschrieben ist, die clearence erheblich gesteigert werden, wenn die Dialysierflüssigkeit durch die Dialysierflüssigkeitskammer des Dialysators rezirkuliert wird. Bei dieser Rezirkulation wird die Umströmung der blutführenden Kapillaren bzw. die Überströmung der Dialysemembran mit zusätzlicher Dialysierflüssigkeit verbessert, wodurch zwar der Konzentrationsgradient verringert, jedoch bei einigen Dialysatoren die Effektivität gesteigert wird. So läßt sich durch eine Erhöhung der Dialysierflüssigkeitsflusses mittels Rezirkulation die Harnstoffclearence bis zu 25 % verbessern.

Insofern kann also auch erfindungsgemäß die Rezirkulation nicht nur zur Luftabtrennung, sondern vielmehr auch zur Verbesserung der Hämodialyse eingesetzt werden.

Weitere Merkmale der Erfindung sind in der nachfolgenden Beschreibung von drei Ausführungsbeispielen erläutert.

Es zeigen:

Fig. 1 eine Prinzipskizze einer ersten Ausführungsform der erfindungsgemäßen Dialysevorrichtung mit stehender Rezirkulationspumpe im Mitstromverfahren,

Fig. 2 die Ausführungsform gemäß Figur 1 bei laufender Rezirkulationspumpe,

Fig. 3 eine Prinzipskizze einer zweiten Ausführungsform bei stehender Rezirkulationspumpe im Gegenstrombetrieb,

Fig. 4 die Ausführungsform gemäß Figur 3 bei laufender Rezirkulationspumpe,

Fig. 5 eine Prinzipskizze einer dritten Ausführungsform bei stehender Rezirkulationspumpe im Gegenstrombetrieb, und

Fig. 6 die Ausführungsform gemäß Figur 5 bei laufender Rezirkulationspumpe.

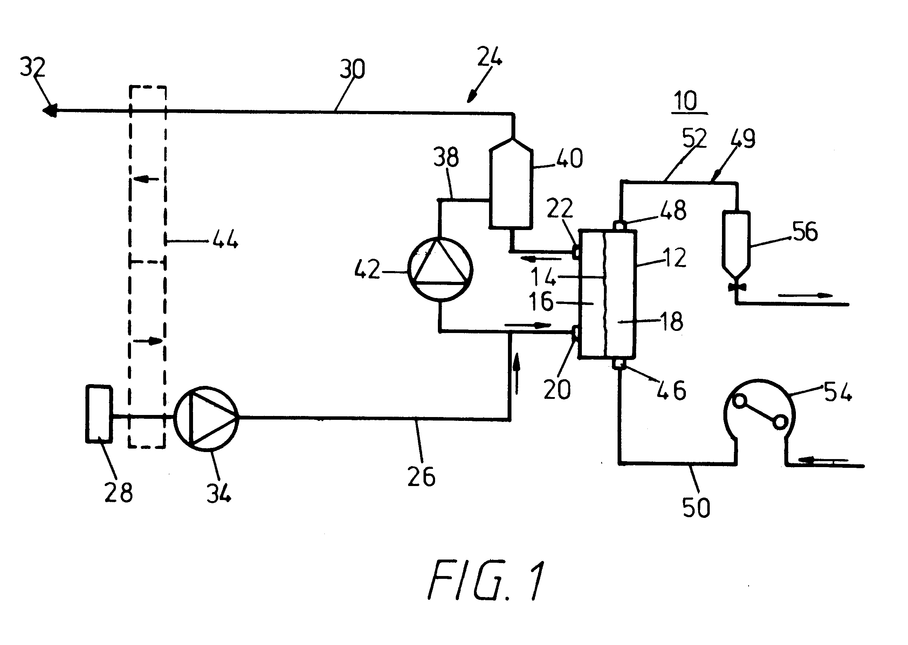

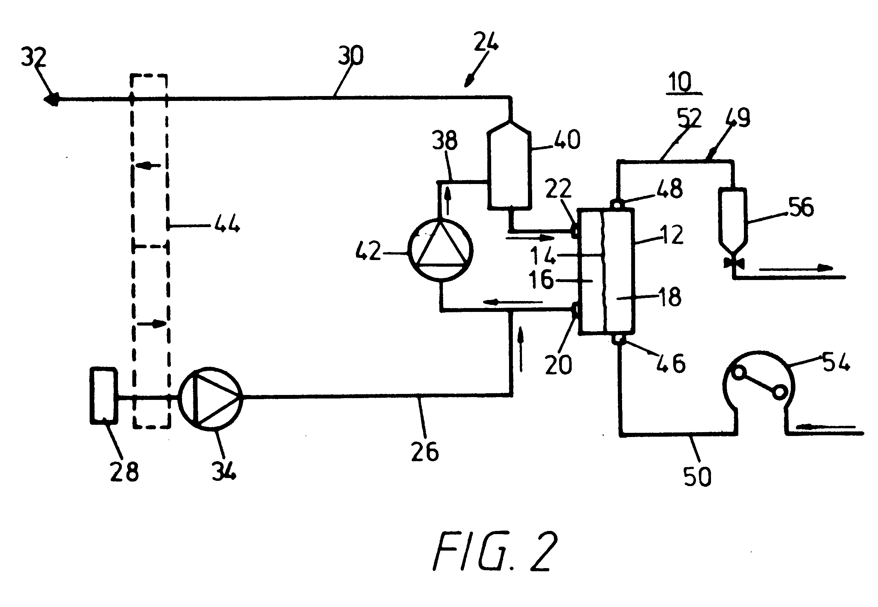

In Figur 1 und 2 ist die Dialysevorrichtung mit 10 bezeichnet. Sie weist einen Dialysator 12 auf, der als Hämodialysator ausgebildet sein kann. Dieser Dialysator 12 ist durch eine Membran 14 in eine Dialysierflüssigkeitskammer 16, die von Dialysierflüssigkeit durchflossen wird, und in eine Blutkammer 18 geteilt, die von Blut durchflossen wird. Die Enden der Dialysierflüssigkeitskammer 16 weisen einen unteren Anschluß 20 und einen oberen Anschluß 22 zum Anschluß an einen Dialysierflüssigkeitsweg 24 auf.

Der Dialysierflüssigkeitsweg 24 besteht aus einer Dialysierflüssigkeitszuleitung 26, die sich von einer Dialysierflüssigkeitsquelle 28 bis zum unteren Anschluß 20 erstreckt. Die Dialysierflüssigkeitsableitung 30 erstreckt sich von dem oberen Anschluß 22 bis zum Abfluß 32.

Der Begriff "unterer Anschluß" und "oberer Anschluß" bedeutet erfindungsgemäß den Zustand des Dialysators 12 und somit der Anschlüsse 20 und 22 im fixierten Einbauzustand, d.h. der obere Anschluß befindet sich im wesentlichen senkrecht über dem unteren Anschluß, so daß eine Strömung der Dialysierflüssigkeit von unten nach oben gegen die Schwerkraft erfolgt.

In den Dialysierflüssigkeitsweg 24 ist eine Dialysierflüssigkeitspumpe 34 eingeschaltet, vorteilhafterweise stromauf des Dialysators 12 in die Dialysierflüssigkeitszuleitung 26. Diese Pumpe 34 kann jedoch aber auch stromab des Dialysators angeordnet sein.

Parallel zum Dialysierflüssigkeitsweg 24 ist eine Rezirkulationsleitung 38 vorgesehen, deren unteres Ende mit dem unteren Anschluß 20 des Dialysators 12 verbunden ist. Sie kann jedoch auch - wie in Figur 1 gezeigt - mit der Dialysierflüssigkeitszuleitung 26 verbunden sein, die - wie vorstehend erläutert - ebenfalls mit dem unteren Anschluß 20 verbunden ist. Beide Anschlußarten haben die gleiche Funktion.

Das obere Ende der Rezirkulationsleitung 38 ist mit einem Luftabscheider 40 verbunden, der in die Dialysierflüssigkeitsableitung 30 eingeschaltet ist und vorteilhafterweise als Behälter ausgebildet ist. In die Rezirkulationsleitung 38 ist weiterhin eine Rezirkulationspumpe 42 eingeschaltet, die von dem unteren Anschluß 20 weg in Richtung Luftabscheider 40 fördert. Im Betriebszustand, in diesem Fall während des Dialysebetriebs, weist die Rezirkulationspumpe 42 eine größere Förderrate als die Dialysierflüssigkeitspumpe 34 auf.

Anstelle einer im offenen Kreislauf betriebenen Hämodialysevorrichtung kann die Ausführungsform gemäß Figur 1 auch als geschlossenes System ausgebildet sein. Hierzu ist - wie in Figur 1 strichliert gezeichnet ist - eine Bilanziereinrichtung 44 vorgesehen, die taktweise betrieben wird und die gleiche Volumina Dialysierflüssigkeit in die Zuleitung 26 und aus der Ableitung 30 des Dialysierflüssigkeitsweges 24 fördert. Ein derartiges System ist in der eingangs erwähnten DE-OS 28 38 414 beschrieben, worauf Bezug genommen wird.

In Figur 1 ist die Hämodialysevorrichtung 10 im Füllbetrieb gezeigt, wie dies durch die Pfeile dargestellt ist, die die strömende Dialysierflüssigkeit andeuten sollen. In der Füllphase steht dabei die Rezirkulationspumpe 42 und bildet somit eine Strömungssperre in der Rezirkulationsleitung 38.

Demgegenüber ist in Figur 2 der Dialysebetrieb dargestellt, wie dies durch die Strömungspfeile ebenfalls ersichtlich ist. Während dieses Dialysebetriebs ist die Rezirkulationspumpe 42 in Betrieb und fördert infolge ihrer gegenüber der Dialysierflüssigkeitspumpe 34 erhöhten Förderrate vom unteren Anschluß 20 die ankommende Dialysierflüssigkeit weg in den Luftabscheider 40, von dessen Bodenbereich ein Teil der Dialysierflüssigkeit zwangsgefördert wird, wobei diese Zwangsförderung zum oberen Anschluß 22 des Dialysators 12 auf die Wirkung der Rezirkulationspumpe 42 zurückzuführen ist.

Der Füll- und Entlüftungsbetrieb wird dabei solange aufrechterhalten, bis ein sicheres Füllen der Kammer 16 mit frischer Dialysierflüssigkeit und eine Entfernung der Luft aus dieser Kammer sichergestellt sind. Anschließend wird die Rezirkulationspumpe 42 in Betrieb genommen, so daß vom Mitstrombetrieb auf den Gegenstrombetrieb übergegangen wird. Zusätzlich erfolgt eine Rezirkulierungsbehandlung, die in der parallelen deutschen Patentanmeldung (P 38 34125), angemeldet am gleichen Tag, beschrieben ist, auf deren Offenbarung Bezug genommen und zum Gegenstand dieser Anmeldung gemacht wird.

Die Blutkammer 18 besitzt ebenfalls einen unteren Anschluß 46 und einen oberen Anschluß 48. Der untere Anschluß 46 ist mit einer Blutzuführungsleitung 50 verbunden, während der obere Anschluß 48 mit einer Blutabführungsleitung 52 verbunden ist. In die Blutzuführungsleitung 50 ist eine Blutpumpe 54 eingeschaltet, die als peristaltische Pumpe ausgebildet ist. Demgegenüber ist in der Blutabführungsleitung 52 eine venöse Tropfkammer 56 üblicher Art eingeschaltet. Der durch die Leitungen 50 und 52 gebildete Blutweg 49 wird auf übliche Weise gefüllt, d.h. die offenen Enden des Blutwegs 49 werden auf übliche Weise mit einem eine physiologische Kochsalzlösung enthaltenden Beutel verbunden, wobei die Lösung solange umgepumpt wird, bis die in dem Blutschlauchsystem und in der Blutkammer 18 enthaltene Luft sicher entfernt ist. Diese Behandlung ist im übrigen in der eingangs genannten DE-OS 34 18 434 beschrieben, auf die Bezug genommen wird.

Bei der in Figur 1 und 2 gezeigten Ausführungsform wird somit während der Fülloperation der Dialysator von unten her mit Lösung gefüllt, wobei mit steigendem Flüssigkeitsspiegel die im Dialysator 12 enthaltende Luft durch die oberen Anschlüsse 22 bzw. 48 in die jeweiligen Abflußleitungen 30 bzw. 52 verdrängt wird. Der Luftabscheider 40 hat dabei nicht nur reine Luftabscheidefunktion, sondern vielmehr auch eine Mischungsfunktion bei der Rezirkulationsbehandlung, die im Dialysierflüssigkeitsbetrieb erfolgt, bei der von einem Mitstrombetrieb (Füllphase) auf einen Gegenstrombetrieb (Dialysebetrieb) übergegangen wird.

Im übrigen ist der Luftabscheider 40 vorteilhafterweise oberhalb des oberen Anschlusses 22 angeordnet, um ein sicheres Abscheiden der Luft zu gewährleisten.

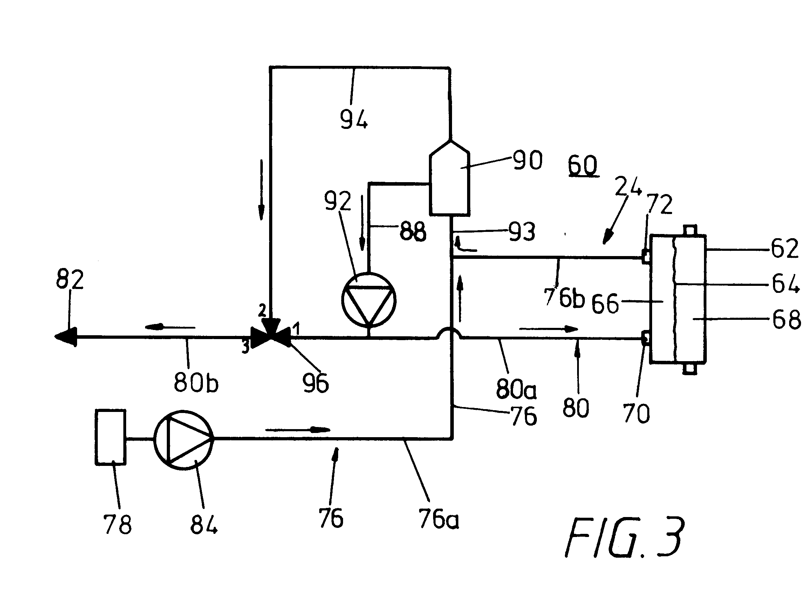

Figur 3 zeigt eine weitere Ausführungsform, die im Dialysebetrieb bei abgestellter Rezirkulationspumpe im Gegenstrom arbeitet.

Aus Übersichtlichkeitsgründen ist der Blutteil der Hämodialysevorrichtung 60 weggelassen worden. Diese Hämodialysevorrichtung 60 weist einen Dialysator 62 auf, der durch eine Membran 64 in eine Dialysierflüssigkeitskammer 16 und eine Blutkammer 18 geteilt ist. Die Dialysierflüssigkeitskammer 16 weist wiederum einen unteren Anschluß 70 und einen oberen Anschluß 72 auf. Durch die Dialysierflüssigkeitskammer 66 ist ein Dialysierflüssigkeitsweg 74 gelegt, der eine Dialysierflüssigkeitszuleitung 76 und eine Dialysierflüssigkeitsquelle 78 aufweist, die mit dem einen Ende der Zuleitung 76 verbunden ist, deren anderes Ende mit dem oberen Anschluß des Dialysators 62 verbunden ist.

Zum Dialysierflüssigkeitsweg 74 gehört weiterhin eine Ableitung 80, die sich vom unteren Anschluß 70 bis zum Abfluß 82 erstreckt. Schließlich ist in die Zuleitung 76 eine Dialysierflüssigkeitspumpe 84 eingeschaltet. Es kann anstelle des auch die in Figur 1 gezeigte Bilanziereinheit verwendet werden, die hier aus Übersichtlichkeitsgründen nicht gezeigt ist.

Eine Rezirkulationsleitung 88 erstreckt sich vom unteren Anschluß 70 oder der Abflußleitung 80 zu einem Luftabscheider 90, der über eine erste Verbindungsleitung 93 mit dem oberen Anschluß 72 bzw. der Zuführungsleitung 76 (unter Auftrennung in die Abschnitte 76a und 76b) des Dialysierflüssigkeitswegs verbunden ist. Bezüglich der Anschlüsse der Rezirkulationsleitung 88 bzw. der Verbindungsleitung 93 an die Anschlüsse 70, 72 bzw. die Leitungen 80, 76 gilt das in der Beschreibung zu Figur 1 Gesagte. Funktionell von Bedeutung ist lediglich der jeweilige Anschluß an den unteren bzw. oberen Teil der Dialysierflüssigkeitskammer bzw. Dialysierflüssigkeitsweg.

Vom oberen Ende des Luftabscheiders 40 geht eine zweite Verbindungsleitung 94 zur Abflußleitung 80 ab, wobei am Verbindungspunkt (Auftrennung der Leitung 80 in die Abschnitte 80a und 80b) mit der Abflußleitung 80 ein Dreiwegeventil 96 als Absperrorgan vorgesehen ist. Dieses Dreiwegeventil 96 verbindet in der Stellung 1-3 den unteren Anschluß mit dem Abfluß 82 und sperrt die zweite Verbindungsleitung 94; sie verbindet andererseits in der Stellung 2-3 den Luftabscheider 90 mit dem Abfluß 82 und sperrt den unteren Anschluß 70 von diesem ab.

Schließlich ist in die Rezirkulationsleitung 88 eine Rezirkulationspumpe 92 eingeschaltet, die von dem Luftabscheider 90 in Richtung auf den unteren Anschluß 70 fördert und deren Förderrate größer ist als diejenige der Dialysierflüssigkeitspumpe 84.

Der Füllbetrieb der zweiten Ausführungsform ist in Figur 3 dargestellt. Hierzu wird die Rezirkulationspumpe 92 betätigt und das Dreiwegeventil 96 in die Stellung 2-3 geschaltet. Aufgrund der größeren Förderrate saugt die Rezirkulationspumpe 92 die ankommende Dialysierflüssigkeit zunächst aus der Zuleitung 76 in die erste Verbindungsleitung 93 und von dort über den Luftabscheider 90 in die Rezirkulationsleitung 88. Sie gelangt anschließend über den Leitungsabschnitt 80a in den unteren Anschluß, wobei die Kammer 66 von unten gefüllt wird. Die verdrängte Luft entweicht über den oberen Anschluß 82 und den Leitungsabschnitt 76b sowie die erste Verbindungsleitung 93 in den Luftabscheider 90. Allmählich wird das gesamte System mit der Dialysierflüssigkeit gefüllt, wobei zu guter Letzt die Luft und anschließend die auszuscheidende Dialysierflüssigkeit über die zweite Verbindungsleitung 94 in den Abfluß 82 verdrängt werden. Der Dialysierflüssigkeitsfluß ist in Figur 3 ebenfalls durch Pfeile gezeigt.

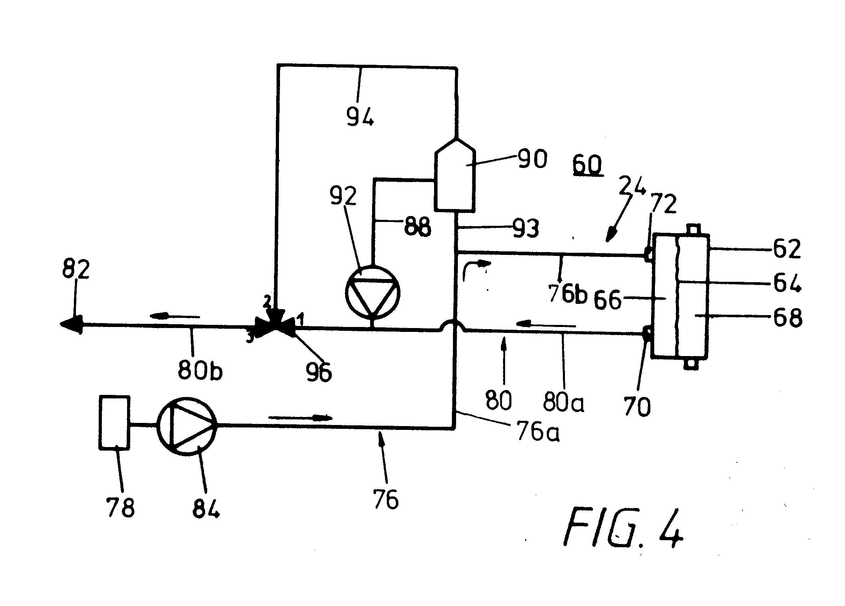

In Figur 4 ist der Dialysierflüssigkeitsbetrieb gezeigt. Zu diesem Zweck wird die Rezirkulationspumpe 92 angehalten. Desgleichen wird das Dreiwegeventil 96 in die Stellung 1-3 geschaltet. Dieses Dreiwegeventil 96 kann im übrigen durch zwei Ventile ersetzt werden, die in den Leitungen 80 und 94 angeordnet sind und wechselweise betätigt werden.

Dabei wird dann die Dialysierflüssigkeitsquelle 78 über die Zuleitung 76 unmittelbar mit dem oberen Anschluß 72 verbunden, während der untere Anschluß 70 unmittelbar mit dem Abfluß 82 verbunden wird. In dieser Phase arbeitet dann die Dialysevorrichtung 60 im Gegenstromprinzip, wie dies vorstehend bereits erläutert worden ist.

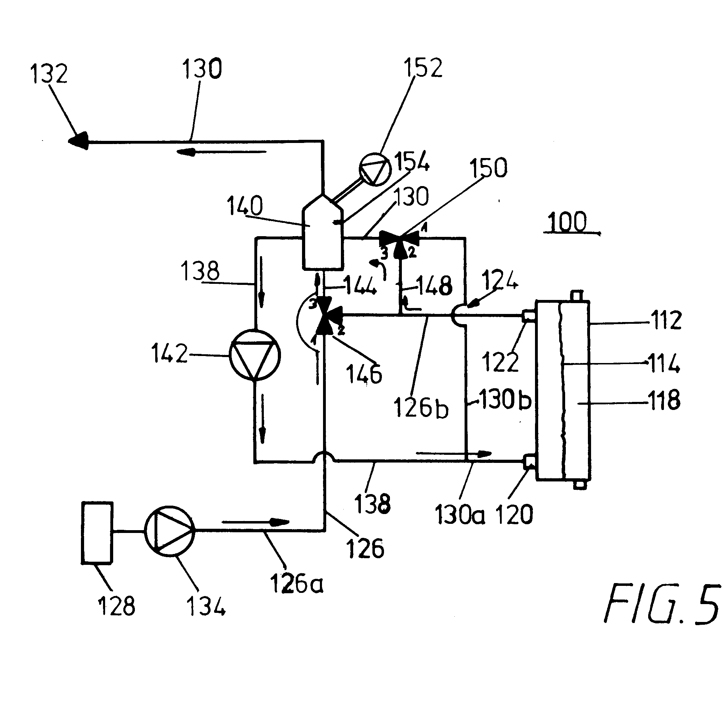

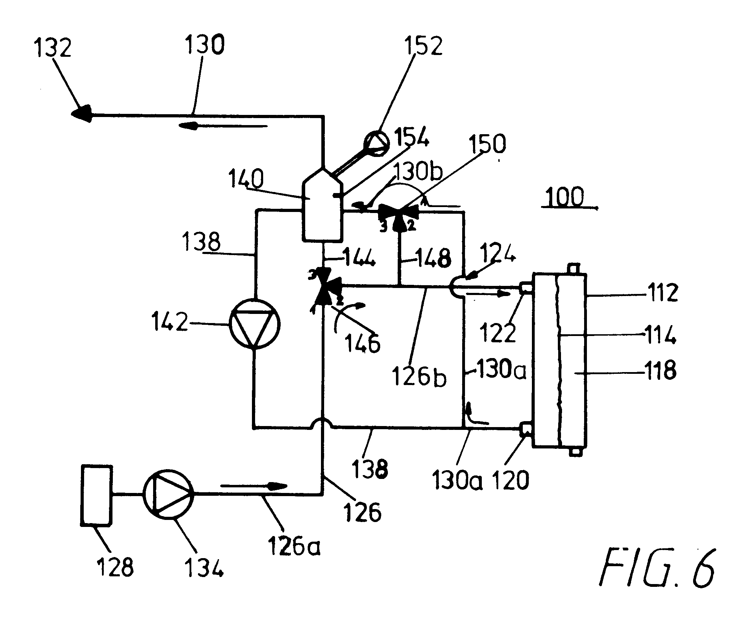

In Figur 5 und 6 ist eine weitere Ausführungsform einer Dialysevorrichtung 110 gezeigt. Diese Vorrichtung 110 weist einen Dialysator 112 auf, der durch eine Membran 114 in eine Dialysierflüssigkeitskammr 116 und in eine Blutkammer 118 geteilt ist. Die Dialysierflüssigkeitskammer 116 weist einen unteren Anschluß 120 und einen oberen Anschluß 122 auf. Durch die Kammer 116 ist ein Dialysierflüssigkeitsweg 124 gelegt, der eine Dialysierflüssigkeitszuleitung 126 aufweist, die an dem einen Ende mit einer Dialysierflüssigkeitsquelle 128 und an dem anderen Ende mit dem oberen Anschluß 120 verbunden ist. Zu dem Dialysierflüssigkeitsweg 124 gehört weiterhin eine Dialysierflüssigkeitsableitung 130, die an dem einen Ende mit dem unteren Anschluß 120 und dem anderen Ende mit dem Abfluß 122 verbunden ist. In die Dialysierflüssigkeitszuleitung 126 ist weiterhin eine Dialysierflüssigkeitspumpe 134 eingeschaltet. Es kann wiederum eine Bilanziereinheit vorgesehen sein, die nicht gezeigt ist und auf deren Beschreibung zu Figur 1 verwiesen wird.

Vom unteren Anschluß 120 bzw. von der Ableitung 130 (unter Auftrennung in die Abschnitte 130a und 130b) geht eine Rezirkulationsleitung 138 ab, deren anderes Ende mit einem Luftabscheider 140 verbunden ist. In diese Rezirkulationsleitung 138 ist eine Rezirkulationspumpe 142 eingeschaltet, die in Richtung auf den unteren Anschluß 120 fördert und die eine größere Förderrate als die Dialysierflüssigkeitspumpe 134 aufweist.

Von der Dialysierflüssigkeitszuleitung 126 geht (unter Auftrennung in die Abschnitte 126a und 126b) eine dritte Verbindungsleitung 144 ab, die im Boden des Luftabscheiders 140 mündet. Am Verbindungspunkt dieser dritten Verbindungsleitung 144 mit der Zuleitung 126 ist ein zweites Dreiwegeventil 146 vorgesehen, das in der 1-2-Stellung die Dialysierflüssigkeitsquelle 128 mit dem oberen Anschluß 122 und in der 1-3-Stellung die Dialysierflüssigkeitsquelle 128 mit dem Luftabscheider 140 verbindet.

Stromab des zweiten Dreiwegeventils 146 geht von dem Zuführungsleitungsabschnitt 126b eine vierte Verbindungsleitung 148 zur Ableitung 130 (Abschnitt 130c) ab. Am Verbindungspunkt mit der Ableitung 130 ist ein drittes Dreiwegeventil 150 vorgesehen, das in der 1-3-Stellung den unteren Anschluß 120 mit dem Luftabscheider 140 verbindet und in der 2-3-Stellung den oberen Anschluß 122 über die Leitung 126b, die Zuleitung 148 und die Ableitung 130c verbindet.

In Figur 5 ist der Füllbetrieb der Dialysevorrichtung 110 gezeigt, wobei das Füllen von unten nach oben erfolgt. Insofern ist die Rezirkulationspumpe 142 in Betrieb genommen. Das zweite Dreiwegeventil 146 befindet sich in der 1-3-Stellung, während das dritte Dreiwegeventil 150 sich in der Stellung 2-3 befindet. Die frische Dialysierflüssigkeit wird zunächst von der Dialysierflüssigkeitspumpe 134 durch die Zuleitung 126a und die dritte Verbindungsleitung 144 in den Luftabscheider 140 gepumpt, von dort durch die Wirkung der Rezirkulationspumpe 142 durch die Rezirkulationsleitung 138 abgepumpt und dem Leitungsabschnitt 130a der Ableitung 130 und somit dem unteren Anschluß 120 zugeführt. Durch die in der Dialysierflüssigkeitskammer 116 ansteigende Dialysierflüssigkeit wird die Luft nach oben durch den oberen Anschluß 122, den Leitungsast 126b, die vierte Verbindungsleitung 148 und den Leitungsast 130c in den Entlüfter 140 und von dort durch die Abflußleitung 130 zum Abfluß 132 verdrängt.

Sobald die Dialysierflüssigkeitsseite gefüllt ist, wird vom Mitstrombetrieb auf den Gegenstrombetrieb umgeschaltet, der von oben nach unten erfolgt. Hierzu wird die Rezirkulationspumpe 142 stillgesetzt. Desgleichen wird das Dreiwegeventil 146 in die Stellung 1-2 und das Dreiwegeventil 150 in die Stellung 1-3 umgeschaltet. Dieser Dialysierflüssigkeitsbetrieb ist in Figur 6 gezeigt, wobei in Figur 5 und Figur 6 jeweils der Dialysierflüssigkeitsfluß durch Pfeile dargestellt ist. Die Dialysierflüssigkeit fließt dann durch die Leitung 126, die Kammer 116 und die Ableitung 130 zum Abfluß 132.

Die in Figur 5 und 6 gezeigte Ausführungsform hat gegenüber der in Figur 3 und 4 gezeigten Ausführungsform den Vorteil, daß in beiden Betriebsphasen (Füll- bzw. Dialysierbetrieb) jeweils die abgeschiedene Luft im Luftabscheider 140 abgetrennt werden kann.

Wird eine Bilanziereinheit verwendet, die exakt bilanziert, so ist vorteilhafterweise in dem Luftabscheider 140 eine Entlüftungspumpe 152 vorgesehen, die auf eine Niveauregulierungseinheit 154 anspricht. Derartige Entlüftungseinrichtungen sind an sich bekannt und werden beispielsweise bei der eingangs erwähnten "A 2008" der Anmelderin verwendet. Als Rezirkulationspumpen können schließlich Zahnradpumpen verwendet werden, wie sie bei den üblichen Dialysierflüssigkeitspumpen zum Einsatz kommen.

**Patentansprüche**

1. Hämodialysevorrichtung (10, 60, 110) mit Entlüftungseinrichtung mit einem Dialysator (12, 62, 112), der durch eine semipermeable Membran (14, 64, 114) in eine Blutkammer (18, 68, 118) und eine Dialysierflüssigkeitskammer (16, 66, 116) geteilt ist, mit einem extrakorporalen Blutweg (49), der durch die Blutkammer (18, 68, 118) gelegt ist, mit einem Dialysierflüssigkeitsweg (24, 74, 124), der durch die Dialysierflüssigkeitskammer (16, 66, 116) gelegt ist eine Dialysierflüssigkeitszuleitung (26, 76, 126), die an dem einen Ende mit einer Dialysierflüssigkeitsquelle (28, 78, 128) und an dem anderen Ende mit dem Einlaß der Dialysierflüssigkeitskammer (16, 66, 116) verbunden ist, und eine Dialysierflüssigkeitsableitung (30, 80, 130), die mit dem Auslaß der Dialysierflüssigkeitskammer (16, 66, 116) verbunden ist, aufweist, während der Blutweg (49) eine Zuführungsleitung (50), die mit dem unten angeordneten Eingang (46) der Blutkammer (18, 68, 118) verbunden und in die eine Blutpumpe (54) eingeschaltet ist, und eine Abführungsleitung (52) aufweist, die mit dem oben angeordneten Auslaß (48) der Blutkammer (18, 68, 118) verbunden ist und die eine venöse Tropfkammer (56) aufweist, mit einer Dialysierflüssigkeitspumpe (34, 84, 134) im Dialysierflüssigkeitsweg (24, 74, 124) und mit einer Luftabscheideeinrichtung (40, 900, 140) im Dialysierflüssigkeitsweg (24, 74, 124), der jeweils stromauf und stromab der Dialysierflüssigkeitskammer (16, 66, 116) des Dialysators (12, 62, 112) mit einer Rezirkulationseinrichtung (38, 88, 138; 42, 92, 142) verbunden ist, welche im Betriebszustand die Strömungsrichtung der Dialysierflüssigkeit durch die Dialysierflüssigkeitskammer (16, 66, 116) umkehrt, dadurch gekennzeichnet, daß die Rezirkulationseinrichtung eine Rezirkulationspumpe (42, 92, 142) ist, welche in einer Rezirkulationsleitung (38, 88, 138) vorgesehen ist und im Betriebszustand eine größere Pumprate aufweist als die Dialysierflüssigkeitspumpe (34, 84, 134) und daß die Luftabscheideeinrichtung (40, 90, 140) zumindest in der Entlüftungsphase in die Dialysierflüssigkeitsableitung (30, 80, 130) geschaltet ist.

2. Vorrichtung nach Anspruch 1, dadurch gekennzeichnet, daß die Dialysierflüssigkeitsleitung (26) an den unteren Anschluß (20) und die Dialysierflüssigkeitsableitung (34) an den oberen Anschluß (22) der Dialysierflüssigkeitskammer (16) angeschlossen sind, daß der Luftabscheider (40) in die Ableitung (24) eingeschaltet ist und daß die Rezirkulationspumpe (42) im Betriebszustand (Dialysebetrieb) vom unteren Anschluß (20) zum oberen Anschluß (22) fördert und im Füllbetrieb stillgesetzt ist.

3. Vorrichtung nach Anspruch 1, dadurch gekennzeichnet, daß die Dialysierflüssigkeitszuleitung (76) an den oberen Anschluß (72) und die Dialysierflüssigkeitsableitung (80) an den unteren Anschluß (70) angeschlossen sind, von der Zuleitung (76) eine erste Verbindungsleitung (93) zum Luftabscheider (90) abgeht, vom Luftabscheider (90) die Rezirkulationsleitung (88) zur Zuleitung (80) abgeht, die Rezirkulationspumpe (92) im Betriebszustand (Füllzustand) vom Luftabscheider (90) auf den unteren Anschluß (70) fördert, vom Luftabscheider (90) eine zweite Verbindungsleitung (94) zur Abflußleitung (80) abgeht und daß der Abflußleitung (80) und der zweiten Verbindungsleitung (94) eine Absperreinrichtung (96) zugeordnet ist, die in der 1-3-Stellung den unteren Anschluß (70) über die Abflußleitung (80) mit dem Abfluß (82) verbindet, während sie in der 2-3-Stellung (Füllbetrieb) den Luftabscheider (90) über die zweite Verbindung (94) mit dem Abfluß (82) verbindet.

4. Vorrichtung nach Anspruch 1, dadurch gekennzeichnet, daß die Dialysierflüssigkeitszuleitung (126) mit dem oberen Anschluß (122) der Dialysierflüssigkeitskammer (116) und die Dialysierflüssigkeitsableitung (130) mit dem unteren Anschluß (120) verbunden sind, von der Dialysierflüssigkeitszuleitung (126) eine dritte Verbindungsleitung (144) zum Luftabscheider (140) abgeht, vom Luftabscheider (140) die Rezirkulationsleitung (138) zur Dialysierflüssigkeitszuleitung (130) oder zum unteren Anschluß (120) abgeht, der Dialysierflüssigkeitszuleitung (126) und der dritten Verbindungsleitung (144) eine zweite Absperreinrichtung (146) zugeordnet ist, die in der 1-2-Stellung die Dialysierflüssigkeitsquelle (128) mit dem oberen Anschluß (122) und in der 1-3-Stellung mit dem Luftabscheider (140) verbindet, stromab der Absperreinrichtung (146) eine vierte Verbindungsleitung von der Dialysierflüssigkeitszuleitung (126) zur Dialysierflüssigkeitsableitung (130) abgeht und dort in einer dritten Absperreinrichtung (150) mündet, die in der 1-3-Stellung (Dialysebetrieb) den unteren Anschluß (120) und in der 2-3-Stellung (Entlüftungsbetrieb) den oberen Anschluß (122) jeweils mit dem Luftabscheider (140) verbindet, und daß die Rezirkulationspumpe (142) im Entlüftungsbetrieb vom Luftabscheider (140) in Richtung unterer Anschluß (120) fördert.

5. Vorrichtung nach Anspruch 1, dadurch gekennzeichnet, daß die Rezirkulationspumpe (42, 92, 142) eine Zahnradpumpe ist.

6. Vorrichtung nach Anspruch 3 oder 4, dadurch gekennzeichnet, daß die Absperreinrichtung (96, 146, 150) durch ein Dreiwegeventil oder durch zwei Einfachventile gebildet ist.

7. Vorrichtung nach einem der Ansprüche 1-6, dadurch gekennzeichnet, daß der Luftabscheider (40, 90, 140) eine Entlüftungspumpe (152) und eine Niveauregeleinrichtung (154) aufweist.

8. Vorrichtung nach einem der Ansprüche 1 bis 7, dadurch gekennzeichnet, daß das Fördern der Dialysierflüssigkeit im Dialysierflüssigkeitsweg (24, 74, 124) durch das System Dialysierflüssigkeitspumpe (34, 84, 134) / Strömungswiderstand (136) oder durch eine Bilanziereinrichtung (44) gesteuert wird.

9. Verfahren zum Entlüften der Dialysierflüssigkeitskammer eines Dialysators, bei dem Dialysierflüssigkeit vom unteren Anschluß des Dialysators zum oberen Anschluß gepumpt wird, wobei die in der Dialysierflüssigkeitskammer enthaltene Luft aus dem oberen Anschluß in den Abfluß verdrängt wird, dadurch gekennzeichnet, daß man den Dialysatorin einer Halteeinrichtung fest anordnet und danach Dialysierflüssigkeit entweder

(a) dem unteren Anschluß mittels einer Dialysierflüssigkeitspumpe zuführt und nach der Entlüftung mit einer parallel zum Dialysierflüssigkeitsweg eröffneten Rezirkulationseinrichtung, die eine größere Pumprate als die Dialysierflüssigkeitspumpe aufweist, dem oberen Anschluß zuführt, so daß der Fluß der Dialysierflüssigkeit durch die Dialysierflüssigkeitskammer umgekehrt wird oder

(b) mittels der Rezirkulationseinrichtung gemäß Variante (a) dem unteren Anschluß und nach dem Entlüften unter Desaktivieren der Rezirkulationseinrichtung dem oberen Anschluß zuführt.

**Claims**

1. A hemodialysis apparatus (10, 60, 110) having a deaeration device, said apparatus comprising

a dialyser (12, 62, 112) subdivided into a blood chamber (18, 68, 118) and a hemodialysis solution chamber (16, 66, 116) by means of a semipermeable membrane (14, 64, 114),

an extracorporal blood stream track (49), conducted through the said blood chamber (18, 68, 118),

a hemodialysis solution track (84, 74, 124) conduted through the hemodialysis solution chamber (16, 66, 116) and comprising a hemodialysis solution feeding tube (26, 76, 126) connected at its one end with the hemodialysis solution source (28, 78, 128) and at its other end with the inlet of the hemodialysis solution chamber (16, 66, 116), and a hemodialysis solution discharging device (30, 80, 130) connected with the outlet of the hemodialysis solution chamber (16, 66, 116),

whilst the blood stream track (49) comprises a feeding tube (50) connected with the inlet (46) of the blood chamber (18, 68, 118) arranged below, into which feeding tube a blood pump (54) is intercalated, and a discharging tube (52) connected with the outlet (48) of the blood chamber (18, 68, 118) arranged at the top and which discharging tube (52) comprises a venous drop chamber (56),

a hemodialysis solution pump (34, 84, 134) in the hemodialysis solution track (24, 74, 124), and

an air separating device (40, 900, 140) in the hemodialysis solution track (24, 74, 124) connected upstream as well as downstream from the hemodialysis solution chamber (16, 66, 116) of the dialyser (12, 62, 112) with a recirculating device (38, 88, 138; 42, 92, 142) that, in the working mode, reverses the flow direction of the hemodialysis solution through the hemodialysis solution chamber (16, 66, 116),

characterized in that

the recirculation device is a recirculation pump (42, 92, 142) provided in a recirculation tube (38, 88, 188), said recirculation pump revealing a greater pumping rate in the working mode than the hemodialysis solution pump (24, 84, 134), and in that the air separating device (40, 90, 140) is engaged into the hemodialyis solution discharging device (30, 80, 130) at least during the stage of deaeration.

2. Apparatus according to claim 1, characterized in that the hemodialysis solution tube (26) is connected to the lower connection (20) and the hemodialysis solution discharging tube (34) to the upper connection (22) of the hemodialysis solution chamber (16),

that the air separator (40) is intercalated into the discharging tube (24) and

that the recirculation pump (42) in the working order (dialysis working mode) delivers from the lower connection (20) in the direction to the upper connection (22) and that it is made idle during the feeding operation.

3. Apparatus according to claim 1, characterized in that the hemodialysis solution feeding tube (76) is connected to the upper connection (72) and the hemodialysis solution discharging tube (80) to the lower connection (70),

a first connection tube (93) runs from the feeding tube (76) to the air separator (90),

the recirculation tube (88) runs from the air separator (90) to the feeding tube (80),

the recirculation pump (92) delivers in the working order (feeding operation) from the air separator (90) in the direction of the lower connection (70),

a second connecting tube (94) runs from the air separator (90) to the discharging tube (80) and

that to the discharging tube (80) and to the second connecting tube (94) a shut-off device (96) is attributed connecting the lower connection (70) with the outlet (82) via the discharging tube (80) in 1-3-position, whilst in 2-3-position (feeding operation), it connects the air separator (90) with the outlet (82) via the second connecting tube

4. Apparatus according to claim 1, characterized in that the hemodialysis solution feeding tube (126) is connected with the upper connection (122) of the hemodialysis solution chamber (116) and the hemodialysis solution discharging tube (130) with the lower connection (120),

a third connecting tube (144) runs from the hemodialysis solution feeding tube (126) to the air separator (140),

the recirculation tube (138) runs to the hemodialysis solution feeding tube (130) or to the lower connection (120),

a second shut-off device (146) is attributed to the hemodialysis solution feeding tube (126) and to the third connecting tube (144), said second shut-off device connecting the hemodialysis solution source (128) with the upper connection (122) in 1-2-position and with the air separator (140) in 1-3-position,

upstream to the shut-off device (146) a fourth connecting tube runs from the hemodialysis solution feeding tube (126) to the hemodialysis solution discharging tube (130) there ending in a third shut-off device (150) connecting each the lower connection (120) in 1-3-position (dialysis working mode) and the upper connection (122) in 2-3-position (deaeration working mode) with the air separator (140), and

the recirculation pump (142) delivers in the deaeration working mode from the air separator (140) in the direction of the lower connection (120).

5. Apparatus according to claim 1, characterized in that the recirculation pump (42, 92, 142) is a gear pump.

6. Apparatus according to claims 3 or 4, characterized in that the shut-off device (96, 146, 150) is formed by a three-way valve or by two simple valves.

7. Apparatus according to anyone of claims 1-6, characterized in that the air separator (40, 90, 140) comprises a deaeration pump (152) and a leveling device (154).

8. Apparatus according to anyone of claims 1-7, characterized in that delivering the hemodialysis solution in the hemodialysis solution track (24, 74, 124) is controlled by the system consisting of a hemodialysis solution pump (34, 84, 134) and a flow risistance (136) or by a balancing device (44).

9. Process for the deaeration of the hemodialysis solution chamber of a dialyser wherein the hemodialysis solution is pumped from the lower connection of the dialyser to the upper collection and, in doing so, the air contained in the hemodialysis solution chamber is displaced from the upper connection,

characterized in that the dialyser is firmly arranged in a holding device and thereafter the hemodialysis solution is either

(a) supplied to the lower connection by means of a hemodialysis solution pump and, after having been deaerated by means of a recirculation device freshly installed in parallel through the hemodialysis solution tube and having a pumping rate exceeding the pumping rate of the hemodialysis solution pump, the said hemodialysis solution is supplied to the upper connection so as to reverse the flow of the hemodialyis solution through the hemodialysis solution chamber, or

(b) the said hemodialysis solution is supplied to the lower connection by means of the recirculation device according to variant (a) and, after having been deaerated under deactivation of the recirculation device, the said hemodialysis solution is supplied to the upper connection.

## Revendications

1. Appareil d'hémodialyse (10, 60, 110) avec un dispositif de dégazage ayant

un dialyseur (12, 62, 112) subdivisé par moyen d'une membrane sémi-perméable (14, 64, 114) en une chambre de sang (18, 68, 118) et en une chambre de bain de dialyse (16, 66, 116)

une voie de flux sanguin extracorporelle (49) posée à travers la chambre de sang (18, 68, 118),

une voie de bain de dialyse (24, 74, 124) posée à travers la chambre de bain de dialyse (16, 66, 116) et comportant

une conduite d'alimentation pour le bain de dialyse (26, 76, 126), ladite conduite d'alimentation étant connectée à une source du bain de dialyse (28, 78, 128) à l'un des bouts et l'autre bout connecté à l'entrée de la chambre de bain de dialyse (16, 66, 116), ladite voie de bain de dialyse comportant de plus une conduite de décharge du bain de dialyse (30, 80, 130) connectée à la sortie de la chambre de bain de dialyse (16, 66, 116),

tandis que la voie de flux sanguin (49) comporte une conduite d'alimentation (50) connectée à l'entrée (46) de la chambre de sang (18, 68, 118) arrangée en bas et dans laquelle conduite une pompe au sang est intercalée, et la voie de flux sanguin (49) comporte une conduite de sortie (52) connectée à la sortie (48) de la chambre de sang (18, 68, 118) arrangée en 'haut, ladite conduite de sortie comportant une chambre-goutte veineuse (56),

l'appareil d'hémodialyse ayant de plus une pompe à bain de dialyse (34, 84, 134) à la voie de bain de dialyse (24, 74, 124) et un dispositif de dégagement d'air (40, 90, 140) à la voie de bain de dialyse (24, 74, 124) connectée à un dispositif de récirculation (38, 88, 138; 42, 92 142), ledit dispositif de récirculation renversant la direction du flux de bain de dialyse à travers la chambre de bain de dialyse (16, 66. 116) en état de marche, **caractérisé en ce que**

le dispositif de récirculation est une pompe à récirculation (42, 92 142) prévue dans une conduite de récirculation (38, 88, 138) et, en état de marche, la pompe de récirculation ayant un capacité de pompage supérieur au capacité de pompage de la pompe de bain de dialyse (34, 84, 134),

et en ce que, pour le moins au stade de dégazage, le dispositif de dégagement d'air (40, 90, 140) est en prise avec la conduite de décharge du bain de dialyse (30, 80, 130).

2. Appareil selon la revendication 1, caractérisé en ce que la conduite du bain de dialyse (26) est connectée au raccordement inférieur (20) et la conduite de décharge du bain de dialyse (34) au raccordement supérieur (22) de la chambre de bain de dialyse (16), le séparateur d'air (40) est intercalée dans la conduite de décharge (24), et en état de marche (activité de dialyse), la pompe de récirculation (42) délivre du raccordement inférieur (20) au raccordement supérieur (22) et qu'elle est met hors service dans l'exercice de remplissage.

3. Appareil selon la revendication 1, caractérisé en ce que la conduite d'alimentation du bain de dialyse (76) est connectée au raccordement supérieur (72) et la conduite de décharge du bain de dialyse (80) au raccordement inférieur, une première conduite de connexion (93) part de la conduite d'alimentation (76) en le séparateur d'air (90) et la conduite de la récirculation (88) part du séparateur d'air (90) en la conduite d'alimentation (80), en état de marche (exercice de remplissage), la pompe de récirculation (92) délivre du séparateur d'air (90) au raccordement inférieur, une seconde conduite de connexion (94) part du séparateur d'air (90) en la conduite de décharge (80), et un dispositif d'arrêt est attribué à la conduite de décharge et à la seconde conduite de connexion (94), ledit dispositif d'arrêt, dans la position 1-3, connectant le raccordement inférieur (70) au déchargeoir (82) par la conduite de décharge (80), pendant que, dans la position 2-3 (exercice de remplissage), il connecte le séparateur d'air (90) au dechargeoir (82) par la seconde connexion (94).

4. Appareil selon la revendication 1, caractérisé en ce que la conduite d'alimentation du bain de dialyse (126) est connecté au raccordement supérieur (122) de la chambre de bain de dialyse (116) et la conduite de décharge du bain de dialyse (130) au raccordement inférieur (120), une troisième conduite de connexion (144) part en le séparateur d'air, la conduite de récirculation ( 138) part du séparateur d'air (140) en la conduite d'alimentation du bain de dialyse (130) ou en le raccordement inférieur (120), un second dispositif d'arrêt (146) est attribué à la conduite d'alimentation du bain de dialyse (126) et à la troisième conduite de connexion (144), ledit dispositif d'arrêt connectant, dans la position 1-2, la source du bain de dialyse (128) au raccordement supérieur (122) et, dans la position 1-3, au séparateur d'air (140), une quatrième conduite de connexion part, en aval du dispositif d'arrêt, de la conduite d'alimentation du bain de dialyse (126) en la conduite de décharge du bain de dialyse (130) y débouchant dans un troisième dispositif d'arrêt (150) qui raccorde au séparateur d'air (140) non seulement le raccordement inférieur (120) dans la position 1-3 (activité de dialyse) mais aussi le raccordement supérieur (122) dans la position 2-3 (exercice de dégazage), et que, dans l'exercice de dégazage , la pompe de récirculation (122) délivre du séparateur d'air (140) dans la direction du raccordement inférieur (120).

5. Appareil selon la revendication 1, caractérisé en ce que la pompe de récirculation (42, 92, 142) est une pompe à engrenages.

6. Appareil selon les revendications 3 ou 4, caractérisé en ce que le dispositif d'arrêt (96, 146, 150) se compose d'une soupape à trois orifices ou de deux soupapes simples.

7. Appareil selon une des revendications 1 à 6, caractérisé en ce que le séparateur d'air (40, 90, 140) comporte une pompe de dégazage (152) et un dispositif de nivelage (154).

8. Appareil selon une des revendications 1 à 7, caractérisé en ce que l'alimentation du bain de dialyse à travers la voie du bain de dialyse (24, 74, 124) est effectuée par le système consistant en la pompe de dialyse (34, 84, 134) et la résistance hydraulique (136) ou par un dispositif équilibreur (44).

9. Procédé pour le dégazage de la chambre de bain de dialyse d'un dialyseur, en quoi le bain de dialyse est pompé du raccordement inférieur du dialyseur au raccordement supérieur, l'air contenu dans la chambre de bain de dialyse étant déplacé du raccordement supérieur dans le déchargeoir, ledit procédé étant

      **caractérisé en ce que**

      le dialyseur est stablement disposé dans un dispositif de retenue, et ensuite on alimente

      ou

(a) le raccordement inférieur au moyen d'une pompe de bain de dialyse et, après le dégazage, on alimente le raccordement supérieur au moyen d'un dispositif à récirculation installé parallelement à la voie du bain de dialyse et présantant un capacité de pompage plus élevé que celui de la pompe de bain de dialyse, si fort que le cours du bain de dialyse est renversé,

      ou

(b) le raccordement inférieur au moyen du dispositif à récirculation selon la variante (a), et après le dégazage, en désactivant le dispositif à récirculation, on alimente le raccordement supérieur.

*FIG. 1*

FIG. 2

EP 0 366 950 B1

FIG. 3

FIG. 4

FIG.5

FIG. 6

EP 0 366 950 B1